# EUROPÄISCHE PATENTSCHRIFT

(11) **EP 1 339 704 B1**
(45) Veröffentlichungstag und Bekanntmachung des Hinweises auf die Patenterteilung: **22.06.2005**
(21) Anmeldenummer: 01989492.2
(22) Anmeldetag: 21.11.2001
(51) Int. Cl.: C07D 311/72

(54) **VERFAHREN ZUR KONTINUIERLICHEN ACYLIERUNG VON CHROMANOLESTERDERIVATEN**
METHOD FOR CONTINUOUSLY ACYLATING CHROMANOL ESTER DERIVATIVES
PROCEDE D'ACYLATION CONTINUE DE DERIVES D'ESTERS DE CHROMANOL

(30) Priorität: 22.11.2000 DE 10058132
(43) Veröffentlichungstag der Anmeldung: 03.09.2003
(73) Patentinhaber: BASF AKTIENGESELLSCHAFT, 67056 Ludwigshafen (DE)
(72) Erfinder: OOST, Carsten, 67098 Bad Dürkheim (DE); KAIBEL, Gerd, 68623 Lampertheim (DE); LAAS, Harald, 67133 Maxdorf (DE); SCHMITT, Peter, 67245 Lambsheim (DE); VON ERDEN, Jens, 67122 Altrip (DE)
(86) Internationale Anmeldenummer: PCT/EP2001/013472
(87) Internationale Veröffentlichungsnummer: WO 2002/042286

(56) Entgegenhaltungen:
- EP-A- 0 784 042
- EP-A- 0 850 937
- WO-A-93/19057
- WO-A-97/28151
- CS-A- 205 951
- DE-A- 2 208 795
- US-A- 3 444 213
- CHEMICAL ABSTRACTS, vol. 82, no. 12, 24. März 1975 (1975-03-24) Columbus, Ohio, US; abstract no. 77109, ONO, SHOJI ET AL: ".alpha.-Tocopherol acetate" XP002197863 in der Anmeldung erwähnt & JP 49 055633 A (TEIJIN LTD.) 30. Mai 1974 (1974-05-30)
- CHEMICAL ABSTRACTS, vol. 102, no. 17, 29. April 1985 (1985-04-29) Columbus, Ohio, US; abstract no. 149553, GABLECH, MILOSLAV ET AL: "Continuous production of d,l-.alpha.-tocopherol acetate" XP002197864 in der Anmeldung erwähnt & CS 205 951 B (CZECH.) 29. Mai 1981 (1981-05-29)

## Beschreibung

Die vorliegende Erfindung betrifft ein Verfahren zur kontinuierlichen Herstellung von Chromanolesterderivaten, insbesondere zur kontinuierlichen Herstellung von Carbonsäureestern der Tocopherole und Tocotrienole durch kontinuierliche Acylierung mit Carbonsäuren oder Carbonsäureanhydriden.

Verbindungen mit Vitamin-E-Aktivität, wie die natürlich vorkommenden Chromanolderivate der Gruppe der Tocopherole und Tocotrienole sind wichtige fett-lösliche Antioxidantien. Ein Mangel an Vitamin E führt bei Menschen und Tieren zu pathophysiologischen Situationen. Vitamin E-Verbindungen haben daher einen hohen wirtschaftlichen Wert als Zusatzstoffe im Food- und Feed-Bereich, in pharmazeutischen Formulierungen und in kosmetischen Anwendungen. Die Verbindungen mit Vitamin E-Aktivität, insbesondere α-Tocopherol, werden dazu hauptsächlich in Form ihrer Acetatester verwendet. Ein wirtschaftliches Verfahren zur Herstellung von Chromanolesterderivaten ist daher von hoher Bedeutung.

Es ist bekannt, Tocopherolderivate diskontinuierlich mit Acetanhydrid zu den entsprechenden Acetatestern umzusetzen.

EP 850 937 beschreibt in den Beispielen ein diskontinuierliches Verfahren zur Herstellung von α-Tocopherolacetat durch Erhitzen von α-Tocopherol mit Essigsäureanhydrid in einem Rührkolben mit aufgesetztem Rückflußkühler unter Rückfluß. Der Reaktionsaustrag wird anschließend destillativ aufgearbeitet.

In DE 19 603 142 wird ein Verfahren zur Herstellung von dl-α-Tocopherol-acetat durch säurekatalysierte Umsetzung von 2,3,5-Trimethylhydrochinon (TMH) mit Phytol oder Isophytol (IP) in einem Lösungsmittel bei erhöhter Temperatur und anschließender Acetylierung des erhaltenen Tocopherols. Die Acetylierung des Tocopherols erfolgt durch säurekatalysierte Umsetzung mit überschüssigem Acetanhydrid. Der Reaktionsaustrag wird durch fraktionierte Destillation im Vakuum aufgearbeitet. Für die kontinuierliche Umsetzung von 2,3,5-Trimethylhydrochinon mit Phytol wird eine Reaktionskolonne vorgeschlagen, in die man eine Mischung aus cyclischem Carbonat, dem Katalysator, TMH und IP seitlich einspeist. Der Kohlenwasserstoff sowie das gebildete Wasser werden am Kopf der Kolonne abgetrennt und aus dem Sumpf heißes cyclisches Carbonat und Vitamin E abgezogen. Die verfahrenstechnische Ausführung der anschließenden Acylierung wird nicht beschrieben. In einem Beispiel wird erwähnt, daß das nach Phasentrennung isolierte Tocopherol mit Acetanhydrid verestert wurde.

Ein Verfahren zur Herstellung von dl-α-Tocopherol bzw. dl-α-Tocopherolacetat durch säurekatalysierte Umsetzung von 2,3,5-Trimethylhydrochinon (TMH) mit Phytol oder Isophytol (IP) in Gegenwart einer Mischung aus ortho-Borsäure und bestimmten aliphatischen Di- oder Tricarbonsäuren und gegebenenfalls anschließender Veresterung mit Acetanhydrid wird in DE 42 08 477 beschrieben. Gemäß DE 42 08 477 wird das hergestellte Tocopherol analog zu DE 19 603 142 diskontinuierlich mit überschüssigem Acetanhydrid säurekatalysiert in Tocopherolacetat überführt und durch fraktionierende Destillation unter stark vermindertem Druck gereinigt. Das molare Einsatzverhältnis Essigsäureanhydrid/Tocopherol betrug über 1,3 mol/mol und die Säurekonzentration ca. 0,055 Mol-% bezogen Tocopherol.

In EP 0 784 042 wird Hydrogen-bis(oxalato)borat als Protonensäure-Katalysator für die Friedel-Crafts-Kondensation von Trimethylhydrochinon mit Isophytol und die Acylierung von Phenolen wie z.B. Tocopherol beansprucht. In einem Beispiel wird die Acylierung von Tocopherol beschrieben. Hierzu wurde Tocopherol mit Essigsäureanhydrid und Hydrogen-bis(oxalato)borat in einem Kolben vorgelegt und das Reaktionsgemisch eine Stunde unter Argon-Atmosphäre zum Rückfluß erhitzt. Das molare Einsatzverhältnis Essigsäureanhydrid/Tocopherol betrug über 1,1 mol/mol und die Boratkonzentration ca. 0,5 Mol-% bezogen Tocopherol. Nach Einengen am Rotationsverdampfer wurde Tocopherolacetat in einer Reinheit von 87 % mit einer Ausbeute von 92 % erhalten. Dieses diskontinuierliche Verfahren weist den Nachteil auf, daß sowohl Ausbeute als auch Reinheit noch nicht zufriedenstellend hoch sind. Zudem ist die Durchführung der Reaktion in einer Argon-Atmosphäre bei einer großtechnischen Herstellung mit hohen Kosten verbunden.

In JP 49 055 633 wird die diskontinuierliche Herstellung von Tocopherolacetat durch Acylierung von Tocopherol mit Acetanhydrid in Gegenwart von anorganischen, festen Säuren, die unlöslich im Reaktionsgemisch sind, beschrieben. Bei dem Verfahren werden Tocopherol und Acetanhydrid im Lösungsmittel Toluol etwa 4 Stunden unter Rückfluß in Gegenwart des Katalysators erhitzt, wobei eine Produktreinheit von etwa 91 % erzielt wird. Als Beispiel für den Katalysator wird SiO₂/Al₂O₃ genannt. Nachteilig an dem Verfahren sind die geringen Raumzeitausbeuten und die geringen Produktreinheiten.

Die Acylierung von Tocopherol mit Essigsäureanhydrid in Gegenwart einer Mischung von Salzsäure und Zink oder Zinkchlorid wird in JP 56 073 081 beschrieben. Nach diesem Verfahren wird Tocopherol mit Essigsäureanhydrid und der Katalysatormischung für 0,5 bis 2 Stunden bei 10 bis 30°C erhitzt. Danach wird der Katalysator abgetrennt und das Reaktionsgemisch mit Wasser gewaschen. Die Salzsäure wird in einer Konzentration von 0,02 bis 0,06 Mol-% bez. Tocopherol, das Zink in einer Konzentration von 0,01 bis 0,2 Mol-% bez. Tocopherol und das Zinkchlorid in einer Konzentration von 0,001 bis 0,1 Mol-% bez. Tocopherol eingesetzt. Das Essigsäureanhydrid wird im 1,2 bis 1,5-fachen molaren Überschuß verwendet. Das Verfahren liefert Tocopherolacetat in einer Ausbeute von 92,5 % bezogen Tocopherol. Die aufwendige Aufarbeitung, die diskontinuierliche Reaktionsführung und das Feststoff-Handling bedingen bei diesem Verfahren trotz der kurzen Reaktionszeit eine geringe Raumzeitausbeute.

In DE 2 208 795 wird die Umsetzung von Trimethylhydrochinon mit Isophytol in Gegenwart einer Mischung von einer Lewis-Säure und einer Protonensäure in einem inerten Lösungsmittel beschrieben. Als Katalysatorsystem kann zum Beispiel eine Mischung von Zinkchlorid mit NaHSO₄, H₂SO₄ oder p-Toluolsulfonsäure eingesetzt werden. Optional kann der Reaktionsaustrag ohne weitere Aufarbeitung mit Essigsäureanhydrid umgesetzt werden. Hierzu wird das Reaktionsgemisch mit Essigsäureanhydrid versetzt und etwa 6 Stunden unter Rückfluß erhitzt. Nachteilig an diesem Verfahren ist die geringe Raumzeitausbeute für die Acylierung.

Ein kontinuierliches Verfahren zur Umsetzung von Trimethylhydrochinon mit Isophytol, Phytol oder Phytadienen in Gegenwart saurer Kondensations-Katalysatoren in einer gepackten Kolonne wird in US 3 444 213 beschrieben. Hierbei werden die Reaktanten, gegebenenfalls vorgemischt oder in einem inerten Lösungsmittel gelöst, auf den Kopf einer beheizten Kolonne gegeben und das entstehende Reaktionswasser über den Kolonnenkopf abgedampft. Bei der Kolonne handelt es sich jedoch lediglich um einen beheizten Rohrreaktor ohne Verdampfer und nicht um eine Reaktivdestillationskolonne. Das am Sumpf der Kolonne anfallende Produkt wird mit Essigsäureanhydrid im Lösungsmittel Pyridin diskontinuierlich innerhalb einer Stunde umgesetzt. Nachteilig am diesem Verfahren ist die geringe Raumzeitausbeute der Acylierung und die Verwendung eines Lösungsmittels.

Ein weiteres kontinuierliches Verfahren zur Umsetzung von Trimethylhydrochinon mit Isophytol wird in CS 205 951 beschrieben. In diesem Patent erfolgt die Acylierung kontinuierlich mit Essigsäureanhydrid in Abwesenheit eines Katalysators.

Alle bekannten Verfahren des Standes der Technik zur Acetylierung von Tocopherolderivaten weisen den Nachteil hoher Verweilzeiten und damit geringer Raum-Zeit-Ausbeuten und hoher Investitionskosten auf. In allen Fällen wird das TocopherolDerivat gegebenenfalls in Gegenwart eines Katalysators diskontinuierlich mit Essigsäureanhydrid umgesetzt und das Reaktionsgemisch destillativ aufgearbeitet. Hierbei werden zunächst die Essigsäure und das Essigsäureanhydrid bei geringem Vakuum abgetrennt und das Acetat des Tocopherolderivats anschließend durch Destillation im Hochvakuum gereinigt.

Weiterhin sind die Ausbeuten dieser Verfahren mit etwa 92 bis 95 % noch nicht befriedigend. Das Essigsäureanhydrid wird stets in relativ hohem Überschuß von mindestens 1,2 mol pro mol Tocopherolderivat eingesetzt, um bei vertretbarer Reaktionszeit einen ausreichenden Umsatz zu erzielen. Wie man EP 0 784 042 entnimmt, ist eine Verminderung des Überschusses an Essigsäureanhydrid nur dann möglich, wenn die Säurekonzentration erheblich erhöht wird. Dies führt jedoch zu einer vermehrten Bildung an Nebenprodukten und somit zu einer verminderten Ausbeute und Produktreinheit.

Der Erfindung lag daher die Aufgabe zugrunde, ein weiteres Verfahren zur Herstellung von Chromanolesterderivaten mit vorteilhaften Eigenschaften zur Verfügung zu stellen, das die Nachteile des Standes der Technik nicht mehr aufweist und die Chromanolesterderivate in hohen Ausbeuten und hohen Raum-Zeit-Ausbeuten liefert.

Dementsprechend wurde ein Verfahren zur kontinuierlichen Herstellung von Chromanolesterderivaten der Formel I gefunden, 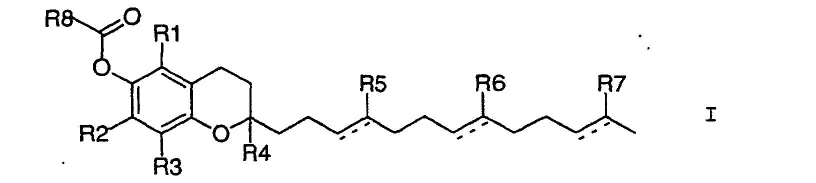 wobei
- R1, R2, R3, R4, R5, R6, R7 und R8: unabhängig voneinander Wasserstoff oder einen gegebenenfalls substituierten, verzweigten oder unverzweigten C₁-C₁₀-Alkylrest und
die gestrichelten Bindungen eine gegebenenfalls zusätzliche C-C-Bindung bedeuten,
indem man kontinuierlich zugeführte Chromanolderivate der Formel II 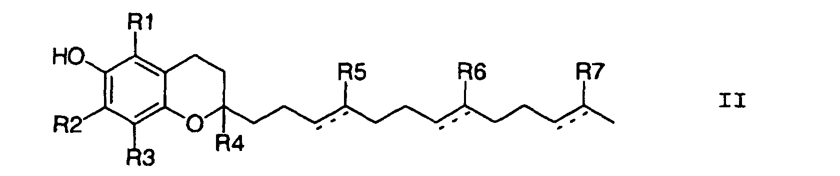 mit kontinuierlich zugeführten Acylierungsmittel, ausgewählt aus der Gruppe Carbonsäuren der Formel IIIa

R8-COOH IIIa

und Carbonsäureanhydriden der Formel IIIb, 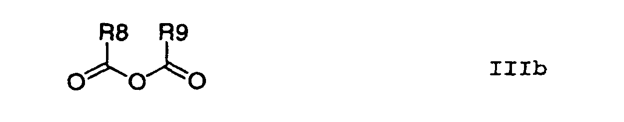 wobei
- R9: Wasserstoff oder einen gegebenenfalls substituierten, verzweigten oder unverzweigten C₁-C₁₀-Alkylrest bedeutet,
in Gegenwart eines Katalysators in einer Reaktionskolonne als Reaktor umsetzt,
die Reaktionsprodukte kontinuierlich aus dem Reaktor ausführt und
bei der Umsetzung mindestens ein Reaktionsprodukt aus dem Reaktionsgemisch abtrennt.

Unter einem gegebenenfalls substituierten, verzweigten oder unverzweigten C₁-C₁₀-Alkylrest werden für die Reste R1, R2, R3, R4, R5, R6, R7, R8 und R9 unabhängig voneinander beispielsweise gegebenenfalls substituiertes Methyl, Ethyl, Propyl, 1-Methylethyl, Butyl, 1-Methylpropyl, 2-Methylpropyl, 1,1-Dimethylethyl, Pentyl, 1-Methylbutyl, 2-Methylbutyl, 1,2-Dimethylpropyl, 1,1-Dimethylpropyl, 2,2-Dimethylpropyl, 1-Ethylpropyl, Hexyl, 1-Methylpentyl, 1,2-Dimethylbutyl, 1,3-Dimethylbutyl, 2,3-Dimethylbutyl, 1,1-Dimethylbutyl, 2,2-Dimethylbutyl, 3,3-Dimethylbutyl, 1,1,2-Trimethylpropyl, 1,2,2-Trimethylpropyl, 1-Ethylbutyl, 2-Ethylbutyl, Heptyl, Octyl, Nonyl oder Decyl, vorzugsweise gegebenenfalls substituiertes C₁-C₄-Alkyl, wie beispielsweise Methyl, Ethyl, iso-Propyl, n-Propyl, n-Butyl, sec-Butyl oder tert-Butyl verstanden.

Die Art der Substituenten ist nicht kritisch. Die C₁-C₁₀-Alkylreste können je nach freien Bindungsmöglichkeiten bis zu 6 Substituenten enthalten, vorzugsweise ausgewählt aus der Gruppe -NO₂, -NH₂, -OH, -CN, -COOH, oder Halogen, insbesondere F oder Cl.

In einer bevorzugten Ausführungsform sind die verzweigten oder unverzweigten C1-C10-Alkylreste der Reste R1, R2, R3, R4, R5, R6, R7, R8 und R9 nicht substituiert.

Besonders bevorzugte Reste für R4, R5, R6 und R7 sind unabhängig voneinander Wasserstoff oder Methyl, insbesondere Methyl.

Besonders bevorzugte Reste für R1, R2 und R3 sind unabhängig voneinander Wasserstoff oder Methyl.

Besonders bevorzugte Reste für R8 sind Methyl oder Ethyl, insbesondere Methyl

Besonders bevorzugte Reste für R9 sind Ethyl oder Methyl.

In einer bevorzugten Ausführungsform des erfindungsgemäßen Verfahrens werden als Chromanolderivate der Formel II Tocopherolderivate und Tocotrienolderivate mit Vitamin-E-Aktivität, insbesondere die natürlich vorkommenden Tocopherole und Tocotrienole verwendet.

Für die bevorzugten, natürlich vorkommenden Tocopherole und Tocotrienole bedeuten in Formel II die Reste R4 bis R7 Methyl. Die Gruppe der Tocopherole (IIa-d) weist eine gesättigte Seitenkette auf, die Gruppe der Tocotrienole (IIe-h) eine ungesättigte Seitenkette: 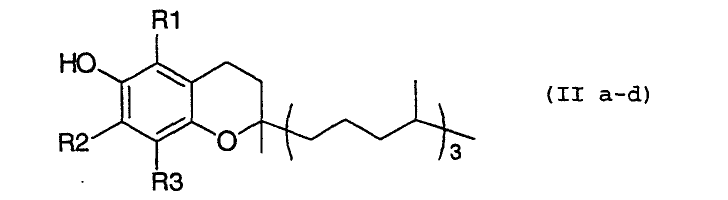
IIa, α-Tocopherol: R1 = R2 = R3 = CH₃
IIb, β-Tocopherol: R1 = R3 = CH₃, R2 = H
IIc, γ-Tocopherol: R1 = H, R2 = R3 = CH₃
IId, δ-Tocopherol: R1 = R2 = H, R3 = CH₃

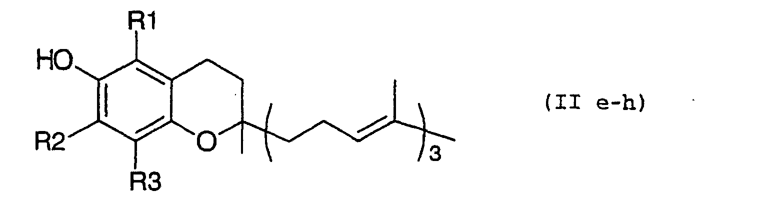

IIe, α-Tocotrienol: R1 = R2 = R3 = CH₃
IIf, β-Tocotrienol: R1 = R3 = CH₃, R2 = H
IIg, γ-Tocotrienol: R1 = H, R2 = R3 = CH₃
IIh, δ-Tocotrienol: R1 = R2 = H, R3 = CH₃

Besonders bevorzugt wird im erfindungsgemäßen Verfahren α-Tocopherol der Formel IIa als Chromanolderivat der Formel II verwendet.

Die im erfindungsgemäßen Verfahren verwendeten Chromanolderivate der Formel II, insbesondere die bevorzugten Tocopherole und Tocotrienole der Formeln IIa bis IIh können als einzelne Verbindungen, die in beliebiger Reinheit vorliegen können, eingesetzt werden. In der Regel liegt die Reinheit einzelner Tocopherole und Tocotrienole bei 90 % bis 97 %, es können aber auch reinere Verbindungen und weniger reine Rohprodukte umgesetzt werden. Die Verbindungen können weiterhin auch als Gemisch verschiedener Chromanolderivate der Formel II eingesetzt werden und das erfindungsgemäße Verfahren führt dementsprechend zu einem Gemisch von Chromanolesterderivaten der Formel I. Dies kann beispielsweise der Fall sein, bei der Verwendung von Tocopherolen oder Tocotrienolen aus natürlichen Quellen ohne weitere Auftrennung der einzelnen Tocopherole und Tocotrienole. Die Chromanolderivate der Formel II können enantiomerenrein, als racemisches Gemisch oder als Diastereomeren-Gemisch vorliegen.

Die Chromanolderivate der Formel II können chemisch hergestellt oder aus natürlichen Quellen, wie beispielsweise dem bei der Pflanzenöldesodorierung anfallenden Dämpferkondensaten isoliert und aufgereinigt werden, wie in Ullmann's Encyclopedia of Industrial Chemistry, Vol. A 27 (1996), VCH Verlagsgesellschaft, Chapter 4., 478-488, Vitamin E, beschrieben.

Die im erfindungsgemäßen Verfahren als Acylierungsmittel eingesetzten Carbonsäuren der Formel IIIa oder Carbonsäureanhydride der Formel IIIb können als Einzelsubstanzen oder als Gemische verwendet werden.

In einer bevorzugten Ausführungsform werden als Acylierungsmittel die Carbonsäureanhydride der Formel IIIb verwendet.

Die Carbonsäureanhydride der Formel IIIb können als reine Carbonsäureanhydride oder als gemischte Carbonsäureanhydride eingesetzt werden. In einer bevorzugten Ausführungsform werden reine Carbonsäureanhydride eingesetzt, so daß R8 = R9 bedeutet. Besonders bevorzugt ist die Acetylierung unter Verwendung von Essigsäureanhydrid (Acetanhydrid) als Carbonsäureanhydrid der Formel IIIb mit R8 = R9 = Methyl.

Im erfindungsgemäßen Verfahren werden die Reaktanten, die Chromanolderivate der Formel II und die Acylierungsmittel, ausgewählt aus der Gruppe Carbonsäure der Formel IIIa und Carbonsäureanhydride der Formel IIIb, kontinuierlich einem Reaktor zugeführt, in dem Reaktor umgesetzt und anschließend die Reaktionsprodukte kontinuierlich aus dem Reaktor entfernt.

Bei Reaktionen mit hoher Anfangsgeschwindigkeit kann es vorteilhaft sein, dem Reaktor einen weiteren Vorreaktor vorzuschalten, in dem bereits ein Teilumsatz stattfindet. Auch kann es vorteilhaft sein, die Reaktanten vor Einspeisung in den Reaktor zu mischen, so daß auch schon hier ein Teilumsatz stattfindet.

Unter dem Begriff "in einem Reaktor umsetzt" wird also verstanden, daß in diesem Reaktor noch ein Umsatz der Reaktanten stattfindet. Dieser Umsatz im Reaktor kann, beispielsweise bei Vorschalten eines Vorreaktors, mindestens 1 %, vorzugsweise mindestens 20 %, besonders bevorzugt mindestens 50 %, ganz besonders bevorzugt mindestens 80 % des insgesamt erreichbaren Umsatzes betragen. In einer bevorzugten Ausführungsform findet der gesamte erreichbare Umsatz im Reaktor statt.

Bei der Zuführung und Umsetzung der Reaktanten können zusätzlich Lösungsmittel verwendet werden. Besonders vorteilhaft läßt sich das Verfahren allerdings ohne Zusatz von Lösungsmitteln durchführen.

Das erfindungsgemäße Verfahren läßt sich besonders vorteilhaft durchführen indem man bei der Umsetzung im Reaktor, also gleichzeitig mit der Umsetzung mindestens ein Reaktionsprodukt aus dem Reaktionsgemisch abtrennt. Dementsprechend wird das aus dem Acylierungsmittel entstehende Wasser (bei Verwendung von Carbonsäuren der Formel IIIa als Acylierungsmittel) bzw. die entstehende Carbonsäure R9-COOH (Bei Verwendung von Carbonsäureanhydriden der Formel IIIb als Acylierungsmittel) oder die entstehenden Chromanolesterderivate der Formel I oder beide bei der Umsetzung aus dem Reaktionsgemisch entfernt.

In einer bevorzugten Ausführungsform erfolgt nur die Abtrennung von Wasser bzw. der Carbonsäure R9-COOH aus dem Reaktionsgemisch. Diese Abtrennung erfolgt bevorzugt ebenfalls kontinuierlich.

In einer besonders bevorzugten Ausführungsform des erfindungsgemäßen Verfahrens erfolgt die Umsetzung in einer Reaktionskolonne als Reaktor.

Wie vorstehend beschrieben, kann es vorteilhaft sein, dieser Reaktionskolonne einen Reaktor (Vorreaktor) vorzuschalten, in dem bereits ein Teil des Umsatzes stattfindet. In einer besonders bevorzugten Ausführungsform erfolgt die Umsetzung in einem Reaktor, insbesondere in einer Reaktionskolonne.

Eine Reaktionskolonne, die auf verschiedenste Art ausgestaltet sein kann, hat die Eigenschaft als Reaktor gleichzeitig eine Umsetzung von Reaktanten und die thermische Abtrennung mindestens eines Reaktionsproduktes zu ermöglichen.

Vorzugsweise besteht die Reaktionskolonne aus einem Sumpf und einem Aufbau der eine Rektifikation ermöglicht, beispielsweise eine Fraktionierkolonne.

In dieser bevorzugten Ausführungsform unter Verwendung einer Reaktionskolonne ist es weiterhin von Vorteil die Reaktionsparameter so einzustellen, daß
A die Umsetzung der Chromanolderivate der Formel II mit dem Acylierungsmittel auf den Einbauten und gegebenenfalls im Sumpf der Reaktionskolonne erfolgt,
B das bei der Umsetzung mit dem Acylierungsmittel entstehende H₂O (Verwendung von Carbonsäuren der Formel IIIa als Acylierungsmittel) oder die entstehende Carbonsäure R9-COOH (Verwendung von Carbonsäureanhydriden der Formel IIIb als Acylierungsmittel) kontinuierlich mit dem Kopfstrom der Reaktionskolonne abgetrennt wird und
C die bei der Umsetzung entstehenden Chromanolesterderivate der Formel I kontinuierlich mit dem Sumpfstrom der Reaktionskolonne abgetrennt werden.

Je nach Ausgestaltungsform der Reaktionskolonne und der verwendeten Reaktanten, wird dies durch unterschiedliche Einstellungen der Reaktionsparameter erreicht. Geeignete Reaktionsparameter sind beispielsweise Temperatur, Druck, Rücklaufverhältnis in der Kolonne, Ausgestaltung der Kolonne, Wärmeführung und Verweilzeit, insbesondere im Sumpf, Energieeintrag oder das Molverhältnis der Reaktanten, die der Fachmann durch Routineversuche so optimieren kann, daß die Merkmal A, B und C erreicht werden.

Typischerweise wird im erfindungsgemäßen Verfahren der Druck am Kolonnenkopf so eingestellt, daß die Temperatur im Sumpf zwischen 100 und 300°C, bevorzugt zwischen 130 und 180°C liegt.

Die Verweilzeit in der Reaktionskolonne beträgt typischerweise zwischen 15 Minuten und 6 Stunden, vorzugsweise zwischen 30 Minuten und 3 Stunden.

Das Einsatzverhältnis der Reaktanten ist nicht kritisch, das MolVerhältnis von Acylierungsmittel, also der Carbonsäuren der Formel IIIa oder der Carbonsäureanhydride der Formel IIIb zu Chromanol-Derivat der Formel II liegt normalerweise zwischen 1,0 und 5,0, vorzugsweise zwischen 1,0 und 1,3.

Das erfindungsgemäße Verfahren läßt sich besonders vorteilhaft durchführen, wenn man die Umsetzung in Gegenwart eines Katalysators durchführt.

Unter Katalysator wird ein Stoff verstanden, der in der Lage ist, die Acylierung von Chromanolderivaten der Formel II zu beschleunigen.

Bevorzugte Katalysatoren sind saure oder basische Acylierungs-Katalysatoren, wie zum Beispiel Schwefelsäure, Phosphorsäure, Salzsäure, Essigsäure, Acetate, Zinkchlorid, Triethylamin, Pyridin, tertiäre Basen, Hydrogen-bis(oxalato)borat, saure oder basische Ionentauscher, Zeolithe, SiO₂/Al₂O₃ oder anorganische feste Säuren.

Besonders bevorzugte Katalysatoren sind homogenene basische oder saure Katalysatoren, insbesondere Schwefelsäure oder Phosphorsäure und heterogene Katalysatoren, insbesondere saure Ionentauscher oder saure Zeolithe, die gezielt in die Reaktionszone eingebracht werden.

Die homogenen Katalysatoren haben den Vorteil, daß sie flüssig in die Fraktionierkolonne gepumpt werden können. Die heterogenen Katalysatoren haben den Vorteil, daß sie nicht zu einer Verunreinigung des Produkts oder einer Verschlechterung der Farbzahl des Produktes bei der Aufarbeitung führen.

Die homogenen Katalysatoren werden vorzugsweise in verdünnter Form eingesetzt. So zum Beispiel werden Schwefelsäure und Phosphorsäure typischerweise in einer Konzentration von 0,01 bis 50 %, bevorzugt in einer Konzentration von 0,1 bis 1,0 % verwendet. Die Menge der homogenen Katalysatoren wird vorzugsweise so bemessen, daß ihre Konzentration zwischen 0,001 bis 1,0 Mol-% bezogen auf das Chromanolderivat der Formel II, bevorzugt zwischen 0,01 und 0,1 Mol-% bezogen auf das Chromanolderivat der Formel II beträgt.

Die heterogenen Katalysatoren werden vorzugsweise in die Einbauten der Fraktionierkolonne integriert.

In einer besonders bevorzugten Ausführungsform des erfindungsgemäßen Verfahrens verwendet man als Einbauten der Reaktionskolonne unterhalb der höchsten Einspeisungsstelle der Reaktanten Kolonnenböden und oberhalb der höchsten Einspeisungsstelle der Reaktanten strukturierte Packungen. Besonders vorteilhafte Kolonnenböden ermöglichen eine hohe Verweilzeit der Flüssigkeit, wobei die Verweilzeit auf den Einbauten der Reaktionskolonne bevorzugt mindestens 30 min beträgt.

Bevorzugte Kolonnenböden sind beispielsweise Ventilböden, bevorzugt Glockenböden oder verwandte Bauarten wie zum Beispiel Tunnelböden oder Thormannböden.

Bevorzugte strukturierte Packungen sind beispielsweise strukturierte Packungen vom Typ melapack^{(R)} (Fa. Sulzer), Bx^{(R)} (Fa. Sulzer), B1^{(R)} (Fa. Montz) oder A3^{(R)} (Fa. Montz) oder Packungen mit vergleichbaren Ausgestaltungen.

In einer weiteren besonders bevorzugten Ausführungsform des erfindungsgemäßen Verfahrens speist man den den höhersiedenden Reaktanten, gegebenenfalls mit dem homogenen Katalysator, oberhalb des tiefer siedenden Reaktanten in die Reaktionskolonne ein.

Besonders vorteilhaft läßt sich das Verfahren durchführen, indem die Wärmezufuhr in die Reaktionskolonne zusätzlich zu einem Verdampfer über an der Reaktionskolonne außen anliegende Wärmeaustauscher oder über sich direkt auf den Kolonnenböden befindlichen Wärmeaustauschern erfolgt.

Vorteilhaft ist weiterhin die Einleitung eines Strippgases, vorzugsweise Stickstoff oder Kohlendioxid, in die Reaktionskolonne. Dadurch wird die Abtrennung von Wasser bzw. der entstehenden Carbonsäure R9-COOH erleichtert.

Weiterhin läßt sich das erfindungsgemäße Verfahren vorteilhaft durchführen, indem man aus dem abgeführten Sumpfstrom in einem nachgeschalteten Verdampfer überschüssiges Acylierungsmittel, also die Carbonsäure der Formel IIIa oder das Carbonsäureanhydrid der Formel IIIb, abtrennt und, gegebenenfalls nach Ausschleusung eines Teilstroms, in die Kolonne zurückführt. Dadurch werden hohe Ausbeuten bezogen auf das Acylierungsmittel (Carbonsäure der Formel IIIa oder Carbonsäureanhydrid der Formel IIIb) erreicht.

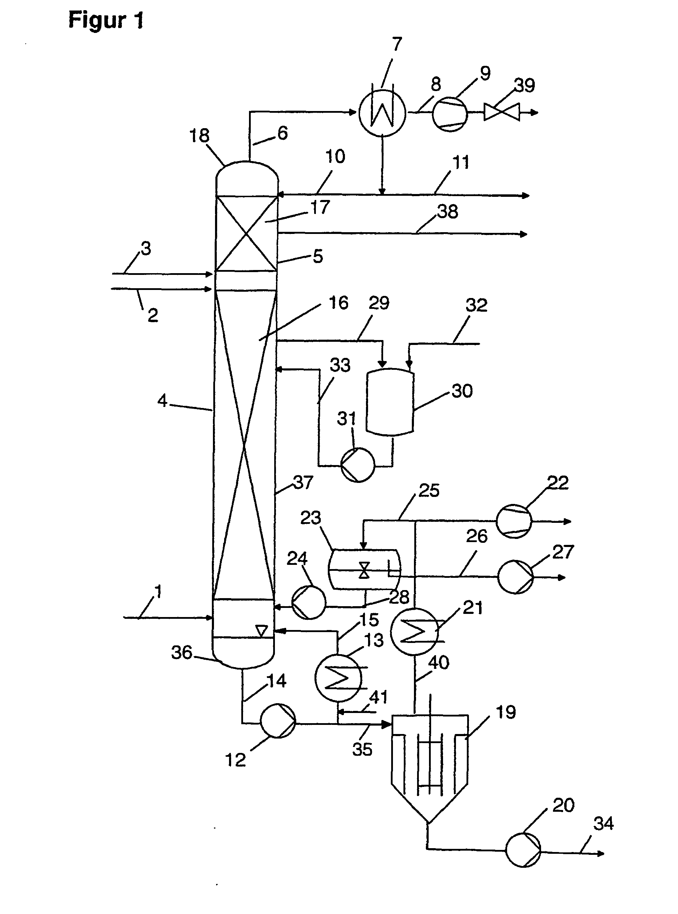

Eine besonders vorteilhafte Ausführungsform des erfindungsgemäßen Verfahrens wird nachfolgend exemplarisch anhand der Figur 1 beschrieben:

Das erfindungsgemäße Verfahren wird vorzugsweise so durchgeführt, daß man das Chromanol-Derivat der Formel II, das Acylierungsmittel (Carbonsäuren der Formel IIIa oder Carbonsäureanhydride der Formel IIIb) und gegebenenfalls den Katalysator über die Zuläufe (3), (1) und (2) auf die Einbauten einer als Reaktionskolonne fungierenden Fraktionierkolonne (4) aufbringt. Dabei ist es von Vorteil, aber nicht zwingend, wenn man den höher siedenden Reaktanten getrennt oder zusammen mit dem Katalysator oberhalb des tiefer siedenden Reaktanten kontinuierlich in die Fraktionierkolonne (4) einspeist. Auf den Einbauten der Fraktionierkolonne findet nun die Umsetzung des Chromanolderivats der Formel II mit dem Acylierungsmittel mit einer überlagerten Destillation statt. Dadurch wird das sich aus der Carbonsäure der Formel IIIa bildende Wasser bzw. die sich aus dem Carbonsäureanhydrid der Formel IIIb bildende Carbonsäure R9-COOH ständig aus dem.Reaktionsgleichgewicht entfernt.

Die Chromanolesterderivate der Formel I gelangen durch die überlagerte Destillation in den Sumpf (36) der Fraktionierkolonne und werden über den Strom (14) abgezogen.

Bei Verwendung von Essigsäureanhydrid oder Propionsäureanhydrid als Carbonsäureanhydrid der Formel IIIb ist es sehr vorteilhaft, wenn man dieses Carbonsäureanhydrid in verdampfter Form in die Kolonne (4) einträgt, da dann eine Spaltung im Sumpf (36) der Kolonne bei hohen Temperaturen und die Bildung von Nebenprodukten weiter reduziert bis vermieden wird.

Weiterhin hat es sich als sehr vorteilhaft erwiesen, wenn die Wärmezufuhr in das Reaktionssystem bestehend aus dem Kolonnensumpf (36) und Fraktionierkolonne (4) und gegebenenfalls Behälter (30) nicht nur über den Verdampfer (13) erfolgt, sondern zusätzlich über außenliegende Wärmetauscher (37) oder über sich direkt auf den Kolonneneinbauten (16) befindliche Wärmetauscher erfolgt.

Die Reaktionskolonne (4) besteht aus zwei Segmenten. Das obere Segment (17) ist vorzugsweise mit strukturierten Packungen gefüllt, das untere Segment (16) vorzugsweise mit Kolonnenböden. Es ist von Vorteil das obere Segment (17) in Abhängigkeit vom Trennaufwand so zu dimensionieren, daß das Acylierungsmittel vollständig zurückgehalten und zur Reaktion in das untere Segment (16) zurückgeführt wird.

Das Wasser bzw. die gebildete Carbonsäure R9-COOH verläßt die Kolonne zusammen mit eventuell in den Reaktanten als Verunreinigung vorhandenen oder während der Reaktion als Nebenprodukt gebildeten Leichtsiedern über den Kopfstrom (6) und gelangt in den Kondensator (7), wo die kondensierbaren Bestandteile dieses Brüdenstroms auskondensiert werden. Ein Teil des Kondensats wird als Rücklauf (10) wieder auf die Kolonne gegeben und der andere Teil (11) abgezogen.

Das Rücklaufverhältnis ist nicht kritisch. Es sollte vorzugsweise ein Rücklaufverhältnis zwischen 1 und 20, besonders bevorzugt zwischen 2 und 4 eingestellt werden. Es ist aber auch möglich, das Kondensat vollständig abzuziehen (Rücklaufverhältnis = 0), wenn der höhere siedende Reaktant am oberen Ende der Kolonne aufgegeben wird.

Das Destillat (11) besteht in der Regel aus Wasser bzw. der Carbonsäure R9-COOH in einer Reinheit von über 99 %. Bei Anwesenheit von leichtsiedenden Verunreinigungen kann es von Vorteil sein, die Carbonsäure R9-COOH in hoher Reinheit über einen zusätzlichen Seitenabzug (38) zu entnehmen. Hierzu muß das obere Kolonnensegment (17) entsprechen dimensioniert werden.

Der Druck am Kolonnenkopf (6) wird vorzugsweise so eingestellt, daß die Temperatur im Sumpf (36) zwischen 100 und 300°C, insbesondere zwischen 130 und 180°C liegt. Je nach Stoffsystem kann dies mit einer Vakuumpumpe (9) und/oder einer Druckregeleinrichtung (39) geschehen.

Das Reaktionsprodukt sammelt sich im Sumpf (36) der Kolonne (4) und wird mittels einer Pumpe (12) zusammen mit den nicht umgesetzten Reaktanten, im wesentlichen zusammen mit überschüssiger Carbonsäure der Formel IIIa bzw. überschüssigem Carbonsäureanhydrid der Formel IIIb, über den Sumpfstrom (14) abgezogen. Ein Teil des Sumpfstroms (14) wird mit einem Verdampfer (13) verdampft und über die Brüdenleitung (15) in die Kolonne geführt. Hierdurch wird der für die Destillation erforderliche Brüden erzeugt. Das Rohprodukt wird über die Produktleitung (35) einem nachgeschalteten Verdampfer (19) zugeführt, mit welchen das Rohprodukt von Leichtsiedern, insbesondere von nicht umgesetzter Carbonsäure der Formel IIIa bzw. nicht umgesetztem Carbonsäureanhydrid der Formel IIIb, befreit wird.

Es ist möglich, zur Entfernung des entstehenden Wassers bzw. der entstehenden Carbonsäure R9-COOH zusätzlich ein Inertgas (41) in den Sumpf der Kolonne einzuspeisen.

Der Verdampfer (19) wird in der Regel mit Hilfe einer Vakuumeinheit (22) bei niedrigerem Druck als die Kolonne (4) betrieben, wobei der Druck so eingestellt wird, daß der Brüdenstrom (40) nur geringe Mengen an Wertprodukt und der Produktstrom (34) nur geringe Mengen an Leichtsiedern enthält. Der Brüdenstrom (40) des Verdampfers (19) wird in einem Kondensator (21) auskondensiert und über die Leitung (25) dem Behälter (23) zugeführt.

Bei Stoffgemischen, bei denen die Carbonsäure der Formel IIIa oder Wasser bzw. das Carbonsäureanhydrid der Formel IIIb oder die Carbonsäure R9-COOH eine Mischungslücke mit den Nebenkomponenten bildet, wird der Behälter (23) als Phasentrenngefäß ausgelegt.

In diesem Fall ist es von Vorteil, die Nebenkomponenten mit Hilfe der Pumpe (27) über die Nebenproduktleitung (26) auszuschleusen und auf diese Weise die Selektivität zu erhöhen sowie die Aufpegelung von Nebenprodukten zu verhindern. Die nicht umgesetzte Reaktanten enthaltene Phase wird mittels einer Pumpe (24) über die Leitung (28) in die Kolonne zurückgeführt. Falls der kondensierte Brüden (25) einphasig ist, so kann es von Vorteil sein, durch Ausschleusung eines Teilstroms über die Leitung (26) eine Aufpegelung von Nebenkomponenten zu verhindern.

Über den Sumpfstrom (34) des Verdampfers (19) wird das von Leichtsiedern befreite Wertprodukt entnommen und gegebenenfalls weiteren Reinigungsstufen wie Kurzwegverdampfern und/oder Molekulardestillation zugeführt.

Die Dosiermengen werden vorzugsweise so gewählt, daß das stöchiometrische Verhältnis von Acylierungsmittel zu dem Chromanol-derivat der Formel II zwischen 1,0 und 5,0, vorzugsweise zwischen 1,0 und 1,3 liegt und sich ein Katalysatorgehalt von 0,001 bis 1,0 Mol-%, vorzugsweise zwischen 0,01 und 0,1 Mol-% bezogen auf das umzusetzende Chromanolderivat einstellt.

Die Verweilzeit des Reaktionsgemisches im Reaktorsystem bestehend aus Sumpf (36) und Fraktionierkolonne (4) und gegebenenfalls Behälter (30) beträgt typischerweise 15 Minuten bis 6 Stunden, vorzugsweise 20 Minuten bis 2 Stunden. Diese Verweilzeit läßt sich vorteilhaft durch Böden (16, 17) mit hoher Verweilzeit der Flüssigkeit erreichen wie beispielsweise Ventilböden, bevorzugt Glockenböden oder verwandte Bauarten wie zum Beispiel Tunnelböden oder Thormannböden. Es ist aber ebenso möglich, Metallgewebepackungen oder Blechpackungen mit geordneter Struktur oder aber Füllkörperschüttungen als Kolonneneinbauten zu verwenden.

Im Segment (17) oberhalb der Zulaufstelle (3) werden vorzugsweise Kolonneneinbauten mit hoher Trennstufenzahl wie Metallgewebepackungen oder Blechpackungen mit geordneter Struktur verwendet.

Weiterhin ist es ebenfalls vorteilhaft, zur Erhöhung der Verweilzeit einen Teilstrom durch einen oder mehrere Seitenabzüge (29) aus der Fraktionierkolonne (4) durch den oder die Behälter (30) zu leiten und mit Hilfe je einer Pumpe (31) die diese Behälter verlassenden Teilströme (33) wieder in die Kolonne (4) zurückzuführen. In die Behälter (30) können mit Hilfe einer Zuleitung (32) gegebenenfalls zusätzlicher Katalysator und/oder Reaktanten dosiert werden.

Die Beheizung der Behälter (30) ist bevorzugt.

Zur Durchführung der Umsetzung verwendet man mit Vorteil Fraktionierkolonnen, die als Einbauten typischerweise 10 bis 100 der oben näher beschriebenen Böden (16, 17), vorzugsweise zwischen 20 und 40 Böden, aufweisen. Hierbei arbeitet man mit Vorteil so, daß man den höher siedenden Reaktanten in den oberen Teil der Kolonne und den niedriger siedenden Reaktanten in den unteren Teil der Kolonne einträgt.

Als besonders vorteilhaft hat es sich erwiesen, wenn in der Kolonne 0 bis 50 Böden, vorzugsweise 5 bis 20 Böden, über dem Zulaufstrom (3) für den höher siedenden Reaktanten und im unteren Teil der Kolonne (16) unterhalb des Zulaufstroms (3) 0 bis 50, vorzugsweise 5 bis 30 Böden, vorgesehen werden.

Die Verweilzeit auf den Einbauten der Kolonne sollte besonders bevorzugt ca. 30 Minuten betragen. Entsprechendes gilt für die sogenannten theoretischen Böden bei anderen Kolonneneinbauten.

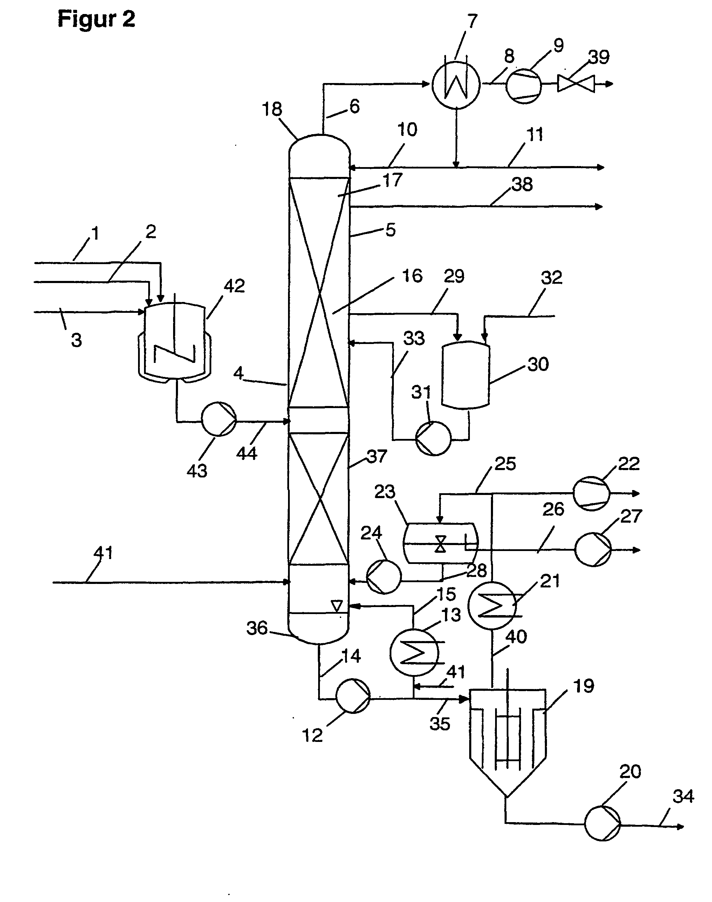

Eine weitere besonders bevorzugte Ausführungsform des erfindungsgemäßen Verfahrens ist in Figur 2 dargestellt:

Insbesondere bei Reaktionen mit hoher Anfangsreaktionsgeschwindigkeit kann es von Vorteil sein, der Reaktionskolonne einen Reaktor (42) vorzuschalten und auf diese Weise die Investitionskosten für die Reaktionskolonne zu reduzieren. Hierzu werden das Chromanol-Derivat der Formel II (3), das Acylierungsmittel (1) und gegebenenfalls der Katalysator (2), gegebenenfalls nach Vorheizung, in den Vorreaktor (42) gepumpt. In diesem Reaktor läuft die Reaktion zunächst mit hoher Anfangsgeschwindigkeit ab.

Im Vorreaktor wird vorzugsweise nur ein Teilumsatz angestrebt und der Austrag des Vorreaktors mit Hilfe der Pumpe (43) über die Leitung (44) in die nachgeschaltete Reaktionskolonne (4) gefördert.

Die Reaktionskolonne (4) wird bei dieser Ausführungsform des erfindungsgemäßen Verfahrens im wesentlichen wie in Figur 1 beschrieben ausgeführt, wobei die Zulaufstelle des Reaktoraustrags (44) in die Kolonne (4) an das jeweilige Stoffsystem und den im Vorreaktor erreichten Umsatz angepaßt werden muß.

Es kann auch von Vorteil sein, nur einen Teil der Gesamtmenge des eingesetzten Acylierungsmittel in den Reaktor (42) zu fördern und den anderen Teil unterhalb der Zulaufstelle für den Reaktoraustrag (44) über eine zusätzliche Leitung (41) in die Reaktionskolonne (4) einzuspeisen. Die Verweilzeit im Vorreaktor wird vorteilhaft so eingestellt, daß die Reaktion im einem Umsatzbereich mit hoher Reaktionsgeschwindigkeit gefahren wird.

In der Regel beträgt die Verweilzeit im vorgeschalteten Reaktor (42) 5 Minuten bis 2 Stunden, bevorzugt 15 Minuten bis 1 Stunde.

Der Reaktor (42) ist in Figur 2 als Rührkessel symbolisiert. In Abhängigkeit des Reaktionssystem sind jedoch auch Reaktoren mit anderen Verweilzeitcharakteristiken wie z.B. Rohrreaktoren oder Schlaufenreaktoren möglich.

Mit Hilfe des erfindungsgemäß verbesserten Verfahrens ist es möglich, zahlreiche Chromanolester-Derivate, insbesondere die als Antioxidans sowie auf dem Gebiet der menschlichen und tierischen Ernährung bedeutenden Tocopherol- und Tocotrienolacetate, insbesondere α-Tocopherolacetat bei nahezu quantitativem Umsatz in sehr hohen Ausbeuten und Raum-Zeit-Ausbeuten sowie hoher Reinheit herzustellen.

Das erfindungsgemäße Verfahren weist gegenüber dem Stand der Technik folgende weitere Vorteile auf:

Mit dem erfindungsgemäßen Verfahren werden Selektivitäten von über 99 % bezogen auf das Chromanol-Derivat und über 90 % bezogen auf das Carbonsäureanhydrid erreicht. Die Selektivität bezogen auf das Carbonsäureanhydrid ist dabei abhängig von der Reinheit des eingesetzten Chromanolderivats, die in den nachstehenden Beispielsen 94 % betrug.

Der Umsatz liegt bei nahezu 100 % bezogen auf das Chromanol-derivat und bei 95 % bezogen auf das Acylierungsmittel, so daß nur geringe Mengen an Acylierungsmittel zurückgeführt werden müssen.

Die Reinheit der Chromanolesterderivate beträgt nach Abtrennung von Carbonsäure der Formel IIIa und Wassser bzw. nach Abtrennung von Carbonsäureanhydrid der Formel IIIb und der Carbonsäure R9-COOH über 95 %. Dieser Wert ist ebenfalls abhängig von der Reinheit des eingesetzten Chromanolderivats der Formel II, da insbesondere bei Tocopherolen und Tocotrienolen als Verunreinigung des Produkts hauptsächlich schon im eingesetzten Tocopherol oder Tocotrienol vorhandene hochsiedende Nebenkomponenten gefunden werden. Nach Abtrennung dieser Komponenten beträgt die Produktreinheit über 99 %, so daß bei dem erfindungsgemäßen Verfahren nur sehr wenig Nebenprodukte gebildet werden.

Ein weiterer Vorteil des Verfahrens ist die kontinuierliche Fahrweise, die eine konstante, nicht chargen-abhängige Produktqualität gewährleistet. Die Raum-Zeit-Ausbeute ist bei vergleichbarer Säurekonzentration um den Faktor 3 größer als bei den bisher bekannten Verfahren. Zudem kann die bei der Reaktion freiwerdende Reaktionswärme zur Destillation genutzt werden und somit Energiekosten eingespart werden.

Ein weiterer großer Vorteil des erfindungsgemäßen Verfahrens.ist, daß die erzielbaren hohen Ausbeuten in einem kontinuierlichen Verfahren in einem Reaktor bei nahezu quantitativem Umsatz bezogen auf alle Einsatzkomponenten erzielt werden, und zwar auch dann, wenn kein Überschuß oder ein nur geringer Überschuß einer der Reaktanten eingesetzt wird.

Weiterhin müssen vorteilhafterweise keine Lösungsmittel verwendet werden und die, bei Verwendung von Carbonsäureanhydriden der Formel III, als Nebenprodukt entstehende Carbonsäure R9-COOH kann als Wertprodukt weiterverwendet werden.

Die nachstehenden Beispiele erläutern die Erfindung

### Beispiel 1:

### Herstellung von α-Tocopherolacetat

### A Beschreibung der Apparatur

Als Apparatur wurde eine Fraktionierkolonne (4) mit 20 Glockenböden (ca. 14 theoretische Stufen) unterhalb der oberen Zulaufstelle (2) und 0,6 Meter einer strukturierten Gewebepackung (Rhombopak 9M) oberhalb der oberen Zulaufstelle (2) verwendet. Die Kolonne hatte einen Innendurchmesser von 30 mm. Die Numerierung der Böden erfolgte von unten nach oben, d.h. der unterste Boden war Boden 1 und der oberste Boden war Boden 20.

Die Kolonne war in regelmäßigen Abständen mit Thermoelementen bestückt, so daß zusätzlich zum Sumpf und zum Kopf der Kolonne an jeder 3. bis 4. theoretischen Stufe die Temperatur gemessen werden konnte. Zusätzlich zum Temperaturprofil konnte mit Hilfe entsprechender Probennahmestellen das Konzentrationsprofil in der Kolonne ermittelt werden.

Der Verdampfer (13), der mit Hilfe eines Thermostaten auf 250°C beheizt werden konnte, hatte ein Volumen von ca. 350 ml, wobei der Füllstand während des Betriebs je nach Verweilzeit zwischen 50 und 155 ml betrug. Auf die Kolonne war ein Kühler aufgesetzt, der mit einem Kryostaten betrieben wurde. Weiterhin war die Kolonne mit einem Vakuumaggregat (9) und einer Kühlfalle ausgestattet. Der Sumpfaustrag (35) der Kolonne wurde auf einen nachgeschalteten Dünnschichtverdampfer (19) geleitet, über dessen Sumpf das von Leichtsiedern befreite Rohprodukt (34) ausgeschleust wurde. Das Destillat des Dünnschichtverdampfers wurde in einem Kondensator (21) kondensiert, in einem Behälter (23) aufgefangen und in die Kolonne zurückgepumpt. Bei Phasenzerfall wurde der Behälter (23) als Trenngefäß verwendet und die untere Phase, die das Carbonsäureanhydrid enthält, in die Kolonne zurückgeführt. Die Apparatur wurde im 24-Stunden-Betrieb gefahren (Stationarität) und alle zu- und ablaufenden Ströme mit Hilfe von Waagen erfaßt und aufgezeichnet.

### B Versuchsdurchführung

### Herstellung von α-Tocopherolacetat ohne Rückführung

Auf den Boden 20 der Kolonne wurden 74,6 g/h (0,16 mol/h) α-Tocopherol (93,6 %ig) sowie 2,05 g/h 1 %ige Schwefelsäure in Essigsäure (0,13 Mol-% Schwefelsäure (100 %) bezogen α-Tocopherol) und auf den Boden 3 der Kolonne 21,.2 g/h (0,34 mol/h) Essigsäureanhydrid gepumpt. Es wurde ein Systemdruck von 400 mbar und ein Rücklaufverhältnis von 2 kg/kg eingestellt.

Die Sumpftemperatur betrug 156°C und die Verweilzeit in dem Reaktorsystem 3 Stunden, die sich aus 1 Stunde auf den Einbauten der Fraktionierkolonne (16) und 2 Stunden im Verdampfer (36) zusammensetzte. Als Sumpfstrom der Kolonne wurden 84,8 g/h Rohprodukt mit 94,0 Gew.-% α-Tocopherolacetat und 0,1 Gew.-% α-Tocopherol gewonnen. Am Kopf der Kolonne wurden 9,6 g/h Destillat bestehend aus 100 Gew.-% Essigsäure abgezogen.

Es wurde α-Tocopherolacetat mit einer Selektivität von 99,9 % bezogen α-Tocopherol und 96,7 % bezogen Essigsäureanhydrid erhalten. Der Umsatz betrug 99,9 % bezogen α-Tocopherol und 84 % bezogen Essigsäureanhydrid. Die Raum-Zeit-Ausbeute betrug etwa 262 g/(1*h).

### Beispiel 2:

### Herstellung von α-Tocopherolacetat ohne Rückführung/Kat in Essigsäureanhydrid (VEA30)

In der in Beispiel 1 beschriebenen Apparatur wurden auf Boden 20 der Kolonne wurden 100,0 g/h (0,22 mol/h) α-Tocopherol (93,6 %ig) sowie 1,9 g/h 1 %ige Schwefelsäure in Essigsäureanhydrid (0,09 Mol-% Schwefelsäure (100 %) bezogen α-Tocopherol) und auf den Boden 3 der Kolonne 26,7 g/h (0,26 mol/h) Essigsäureanhydrid gepumpt. Es wurde ein Systemdruck von 400 mbar und ein Rücklaufverhältnis von 2 kg/kg eingestellt. Die Sumpf temperatur betrug 155°C und die Verweilzeit in dem Reaktorsystem 2,2 Stunden, die sich aus 0,7 Stunden auf den Einbauten der Fraktionierkolonne (16) und 1,5 Stunden im Verdampfer (36) zusammensetzte. Als Sumpfstrom der Kolonne wurden 113,8 g/h Rohprodukt mit 93,7 Gew.-% α-Tocopherolacetat und 0,07 Gew.-% α-Tocopherol gewonnen. Am Kopf der Kolonne wurden 11,6 g/h Destillat bestehend aus 100 Gew.-% Essigsäure abgezogen. Es wurde α-Tocopherolacetat mit einer Selektivität von 99,9 % bezogen α-Tocopherol und 97,9 % bezogen Essigsäureanhydrid erhalten. Der Umsatz betrug 99,9 % bezogen α-Tocopherol und 82 % bezogen Essigsäureanhydrid. Die Raum-Zeit-Ausbeute betrug etwa 350 g/(l*h).

### Beispiel 3:

### Versuchsdurchführung - Herstellung von α-Tocopherolacetat mit Rückführung (VEA53)

Auf den Boden 20 der Kolonne wurden 150,0 g/h (0,33 mol/h) α-Tocopherol (94,9 %ig) sowie 2,1 g/h 1 %ige Schwefelsäure in Essigsäureanhydrid (0,067 Mol-% Schwefelsäure (100 %) bezogen α-Tocopherol) und auf den Boden 3 der Kolonne 35,0 g/h (0,34 mol/h) Essigsäureanhydrid gepumpt. Es wurde ein Systemdruck von 400 mbar und ein Rücklaufverhältnis von 2 kg/kg eingestellt. Die Sumpftemperatur betrug 164°C und die Verweilzeit in dem Reaktorsystem 1,2 Stunden, die sich aus 0,7 Stunden auf den Einbauten der Fraktionierkolonne (16) und 0,5 Stunden im Verdampfer (36) zusammensetzte. Der Sumpfstrom der Kolonne wurde auf den Dünnschichtverdampfer geleitet, das Destillat auskondensiert und auf Boden 3 in die Kolonne zurückgeführt (Rückführstrom 2,2 g/h). Als Sumpfstrom des Dünnschichtverdampfers wurden 164,0 g/h Rohprodukt mit 94,15 Gew.-% α-Tocopherolacetat und 0,34 Gew.-% α-Tocopherol gewonnen. Am Kopf der Kolonne wurden 16,4 g/h Destillat bestehend aus 100 Gew.-% Essigsäure abgezogen.

Es wurde α-Tocopherolacetat mit einer Selektivität von 99,4 % bezogen α-Tocopherol und 91,0 % bezogen Essigsäureanhydrid erhalten. Der Umsatz betrug 99,6 % bezogen α-Tocopherol und 100 % bezogen Essigsäureanhydrid. Die Raum-Zeit-Ausbeute betrug etwa 700 g/(l*h).

### Vergleichsbeispiel 1

### Diskontinuierliche Reaktionsführung

Der diskontinuierliche Vergleichsversuch wurde in einem beheizten Rundkolben mit Rührer, Thermometer und aufgesetztem Rückflußkühler durchgeführt. 300,75 g α-Tocopherol (93,6 %ig) (0,655 mol ber. 100 %) wurden im Kolben vorgelegt und auf 100°C aufgeheizt. Anschließend wurden 86,4 g (0,847 mol) Essigsäureanhydrid mit 0,03 g (0,00031 mol) Schwefelsäure (96 %ig) versetzt und unter Rühren in den Kolben gegeben. Hierbei heizte sich das Reaktionsgemisch durch die freiwerdende Reaktionswärme auf und wurde anschließend auf 140°C geregelt. Nach 2 Stunden wurde das Reaktionsgemisch auf Raumtemperatur abgekühlt. Der Reaktionsaustrag wurde mittels GC analysiert. Im Reaktionsaustrag wurden 299,7 g α-Tocopherolacetat, 0,05 g α-Tocopherol, 44,3 g Essigsäure und 10,2 g Essigsäureanhydrid gefunden. Es wurde α-Tocopherolacetat mit einer Selektivität von 96,9 % bezogen α-Tocopherol und 85,0 % bezogen Essigsäureanhydrid erhalten. Der Umsatz betrug 99,9 % bezogen α-Tocopherol und 88 Gew.-% bezogen Essigsäureanhydrid. Die Gesamtchargenzeit betrug etwa 3 Stunden.

## Patentansprüche

1. Verfahren zur kontinuierlichen Herstellung von Chromanol-esterderivaten der Formel I, 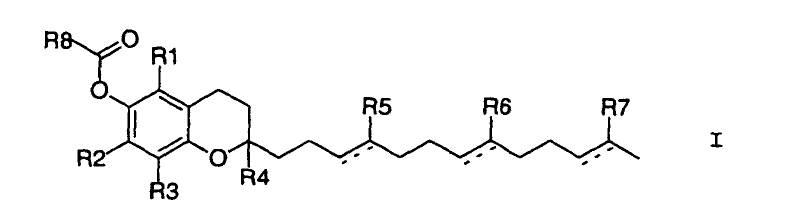 wobei
R1, R2, R3, R4, R5, R6, R7 und R8 unabhängig voneinander Wasserstoff oder einen, gegebenenfalls substituierten, verzweigten oder unverzweigten C₁-C₁₀-Alkylrest und
die gestrichelten Bindungen eine gegebenenfalls zusätzliche C-C-Bindung bedeuten,
indem man kontinuierlich zugeführte Chromanol-Derivate der Formel II 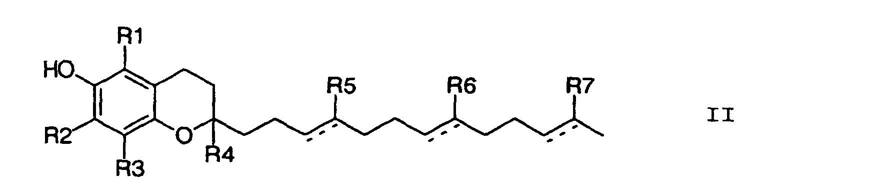 mit kontinuierlich zugeführten Acylierungsmittel, ausgewählt aus der Gruppe Carbonsäuren der Formel IIIa
R8-COOH IIIa
und Carbonsäureanhydriden der Formel IIIb, 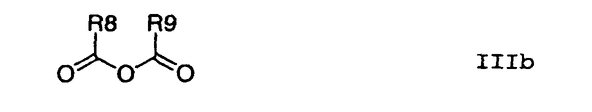 wobei
R9 Wasserstoff oder einen gegebenenfalls substituierten, verzweigten oder unverzweigten C₁-C₁₀-Alkylrest bedeutet,
in Gegenwart eines Katalysators in einer Reaktionskolonne als Reaktor umsetzt,
die Reaktionsprodukte kontinuierlich aus dem Reaktor ausführt und
bei der Umsetzung mindestens ein Reaktionsprodukt aus dem Reaktionsgemisch abtrennt.

2. Verfahren nach Anspruch 1, **dadurch gekennzeichnet, daß** man die Reaktionsparameter so einstellt, daß
A die Umsetzung der Chromanol-Derivate der Formel II mit dem Acylierungsmittel auf den Einbauten und gegebenenfalls im Sumpf der Reaktionskolonne erfolgt,
B das bei der Umsetzung mit den Carbonsäuren der Formel IIIa entstehende H₂O oder die bei der Umsetzung mit den Carbonsäureanhydriden der Formel IIIb entstehende Carbonsäure R9-COOH kontinuierlich mit dem Kopfstrom der Reaktionskolonne abgetrennt wird und
C die bei der Umsetzung entstehenden Chromanolester-Derivate der Formel I kontinuierlich mit dem Sumpfstrom der Reaktionskolonne abgetrennt werden.

3. Verfahren nach Anspruch 1 oder 2, **dadurch gekennzeichnet, daß** man der Reaktionskolonne einen weiteren Reaktor vorausschaltet.

4. Verfahren nach einem der Ansprüche 1 bis 3, **dadurch gekennzeichnet, daß** man als Einbauten der Reaktionskolonne unterhalb der höchsten Einspeisungsstelle der Reaktanten Kolonnenböden und oberhalb der höchsten Einspeisungsstelle der Reaktanten strukturierte Packungen verwendet.

5. Verfahren nach einem der Ansprüche 1 bis 4, **dadurch gekennzeichnet, daß** man den höhersiedenden Reaktanten, gegebenenfalls mit dem Katalysator, oberhalb des tiefer siedenden Reaktanten in die Reaktionskolonne einspeist.

6. Verfahren nach einem der Ansprüche 1 bis 5, **dadurch gekennzeichnet, daß** die Wärmezufuhr in die Reaktionskolonne zusätzlich zu einem Verdampfer
über an der Reaktionskolonne außen anliegende Wärmeaustauscher oder
über sich direkt auf den Kolonnenböden befindlichen Wärmeaustauscher erfolgt und
bei Anwesenheit eines Vorreaktors gemäß Anspruch 3, zusätzlich eine Wärmezufuhr in diesen Vorreaktor erfolgt.

7. Verfahren nach einem der Ansprüche 1 bis 6, **dadurch gekennzeichnet, daß** man ein Strippgas in die Reaktionskolonne einleitet.

8. Verfahren gemäß einem der Ansprüche 1 bis 7, **dadurch gekennzeichnet, daß** man aus dem abgeführten Sumpfstrom in einem nachgeschalteten Verdampfer überschüssiges Acylierungsmittel abtrennt und, gegebenenfalls nach Ausschleusung eines Teilstroms, in die Kolonne zurückführt.

## Claims

1. A process for continuously preparing chromanol ester derivatives of the formula I, 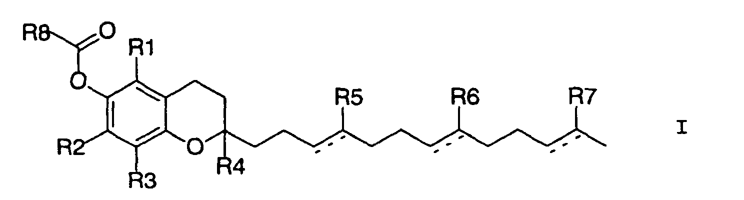 where
R1, R2, R3, R4, R5, R6, R7 and R8 independently of one another are hydrogen or an unsubstituted or substituted , branched or unbranched C₁-C₁₀-alkyl radical and
the dashed bonds are a possible additional C-C bond,
by reacting continuously fed chromanol derivatives of the formula II 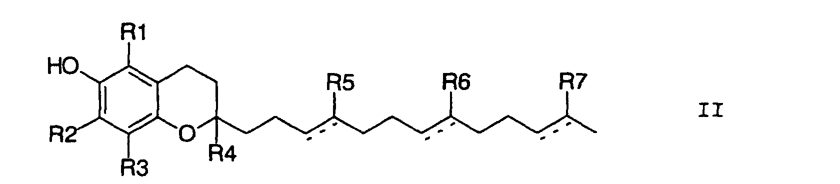 with continuously fed acylating agent selected from the group consisting of carboxylic acids of the formula IIIa
R8-COOH IIIa
and carboxylic anhydrides of the formula IIIb, 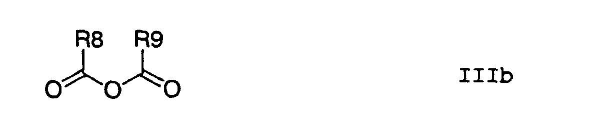 where
R9 is hydrogen or an unsubstituted or substituted, branched or unbranched C₁-C₁₀-alkyl radical,
in the presence of a catalyst in a reaction column as a reactor,
continuously removing the reaction products from the reactor and removing at least one reaction product from the reaction mixture during the reaction.

2. A process as claimed in claim 1, wherein the reaction parameters are set in such a manner that
A the chromanol derivatives of the formula II react with the acylating agent on the internals and possibly in the bottom phase of the reaction column,
B the H₂O formed in the reaction with the carboxylic acids of the formula IIIa or the carboxylic acid R9-COOH formed in the reaction with the carboxylic anhydrides of the formula IIIb is continuously removed with the overhead stream of the reaction column and
C the chromanol ester derivatives of the formula I formed in the reaction are continuously removed with the bottom stream of the reaction column.

3. A process as claimed in claim 1 or 2, wherein a further reactor is connected upstream of the reaction column.

4. A process as claimed in one of claims 1 to 3, wherein the reaction column internals are column trays below the highest feed point for the reactants, and are structured packings above the highest feed point for the reactants.

5. A process as claimed in one of claims 1 to 4, wherein the higher-boiling reactant, with or without the catalyst, is fed into the reaction column above the lower-boiling reactant.

6. A process as claimed in one of claims 1 to 5, wherein heat is fed into the reaction column, in addition to an evaporator
via heat exchangers mounted externally to the reaction column or
via heat exchangers situated directly on the column trays and
when a preliminary reactor as claimed in claim 3 is present, heat is additionally supplied to this preliminary reactor.

7. A process as claimed in one of claims 1 to 6, wherein a stripping gas is introduced into the reaction column.

8. A process as claimed in one of claims 1 to 7, wherein excess acylating agent is removed from the effluent bottom stream in a downstream evaporator and recirculated to the column with or without ejection of a substream.

## Revendications

1. Procédé de préparation continue de dérivés d'ester de chromanol de la formule I : 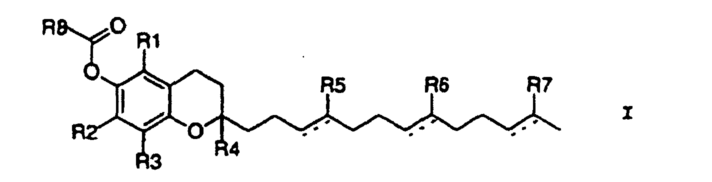 dans laquelle
R1, R2, R3, R4, R5, R6, R7 et R8 représentent, indépendamment, l'un de l'autre, de l'hydrogène ou un radical alkyle en C₁-C₁₀ ramifié ou non ramifié, éventuellement substitué, et
les liaisons en traits interrompus représentent une liaison C-C éventuellement supplémentaire,
dans lequel on fait réagir, dans une colonne réactionnelle comme réacteur, en présence d'un catalyseur, des dérivés de chromanol de la formule II, alimentés de manière continue, 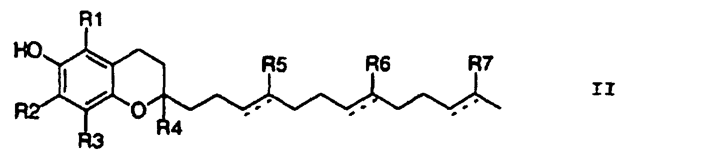 avec des agents d'acylation alimentés de manière continue, choisis parmi le groupe des acides carboxyliques de la formule IIIa :
R8-COOH IIIa
et des anhydrides carboxyliques de la formule IIIb : 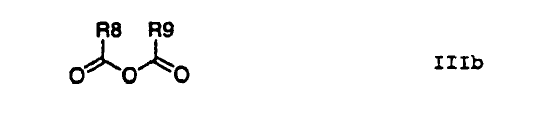 où
R9 représente de l'hydrogène ou un radical alkyle en C₁-C₁₀ ramifié ou non ramifié, éventuellement substitué,
on évacue en continu du réacteur les produits réactionnels, et
au cours de la réaction on isole au moins un produit réactionnel à partir du mélange réactionnel.

2. Procédé suivant la revendication 1, **caractérisé en ce qu'**on ajuste les paramètres de la réaction de façon que
A la réaction des dérivés de chromanol de la formule II avec l'agent d'acylation ait lieu sur les éléments encastrés et éventuellement dans le fond de la colonne de réaction,
B l'H₂O formé au cours de la réaction avec les acides carboxyliques de la formule IIIa ou l'acide carboxylique R9-COOH formé au cours de la réaction avec les anhydrides carboxyliques de la formule IIIb soit isolé de manière continue avec le courant de tête de la colonne réactionnelle, et
C les dérivés d'ester de chromanol de la formule I, formés au cours de la réaction, soient isolés de manière continue avec le courant du fond de la colonne réactionnelle.

3. Procédé suivant l'une des revendications 1 et 2, **caractérisé en ce qu'**on monte un autre réacteur en amont de la colonne réactionnelle.

4. Procédé suivant l'une des revendications 1 à 3, **caractérisé en ce que**, comme éléments encastrés de la colonne réactionnelle, on utilise en dessous de l'emplacement d'alimentation le plus élevé des réactifs des plateaux et au-dessus de l'emplacement d'alimentation le plus élevé des réactifs des garnissages structurés.

5. Procédé suivant l'une des revendications 1 à 4, **caractérisé en ce que**, dans la colonne réactionnelle, on alimente le réactif à point d'ébullition supérieur, éventuellement avec le catalyseur, au-dessus du réactif à point d'ébullition inférieur.

6. Procédé suivant l'une des revendications 1 à 5, **caractérisé en ce que** l'apport de chaleur dans la colonne réactionnelle a lieu en supplément à un évaporateur
par l'intermédiaire d'un échangeur de chaleur extérieur voisin de la colonne réactionnelle, ou
par l'intermédiaire d'un échangeur de chaleur qui se trouve directement sur les plateaux de la colonne, et
en présence d'un réacteur amont conforme à la revendication 3, un apport de chaleur a lieu en supplément dans ce réacteur amont.

7. Procédé suivant l'une des revendications 1 à 6, **caractérisé en ce qu'**on introduit un gaz de stripage dans la colonne réactionnelle.

8. Procédé suivant l'une des revendications 1 à 7, **caractérisé en ce que**, à partir du courant de fond de colonne évacué, on isole dans un évaporateur monté en aval l'excédent d'agent d'acylation et on le recycle dans la colonne, éventuellement après séparation par éclusage d'un courant partiel.
